(19) Europäisches Patentamt
European Patent Office
Office européen des brevets

(11) Veröffentlichungsnummer: 0 019 157
A1

# (12) EUROPÄISCHE PATENTANMELDUNG

(21) Anmeldenummer: 80102377.1

(22) Anmeldetag: 02.05.80

(51) Int. Cl.³: C 07 D 223/20

(30) Priorität: 10.05.79 DE 2918832

(43) Veröffentlichungstag der Anmeldung: 26.11.80 Patentblatt 80/24

(84) Benannte Vertragsstaaten: AT BE CH DE FR GB IT LI LU NL SE

(71) Anmelder: BASF Aktiengesellschaft
Carl-Bosch-Strasse 38
D-6700 Ludwigshafen(DE)

(72) Erfinder: Steiner, Gerd, Dr.
Oberer Waldweg 1
D-6719 Kirchheim(DE)

(72) Erfinder: Franke, Albrecht, Dr.
Mandelring 11
D-6706 Wachenheim(DE)

## (54) 5,6-Dihydro-11-alkylen-morphantridin-6-one und Verfahren zu ihrer Herstellung.

(57) 5,6-Dihydro-11-alkylen-morphantridin-6-one der allgemeinen Formel I

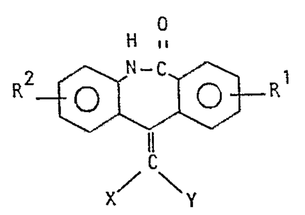

I

in der R¹, und R² Wasserstoffatome, Fluor- oder Chloratome, Methyl oder Trifluormethyl, X ein Wasserstoffatom oder Methyl und Y eine Cyanogruppe, Methylcarbonyl, eine Carbonamidgruppe, gegebenenfalls am Amidstickstoff durch ein oder zwei niedere Alkylreste mit 1 bis 3 C-Atomen substituiert, oder eine Alkoxycarbonylgruppe mit 1 bis 3 C-Atomen im Alkyl bedeuten, die wertvolle Zwischenprodukte für chemische Synthesen, insbesondere für Arzneimittel, darstellen.

EP 0 019 157 A1

5,6-Dihydro-11-alkylen-morphantridin-6-one und Verfahren zu ihrer Herstellung

Die Erfindung betrifft 5,6-Dihydro-11-alkylen-morphantridin-6-one der allgemeinen Formel I

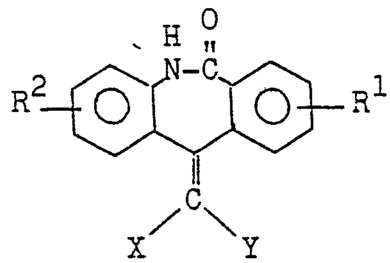

I

in der $R^1$, und $R^2$ Wasserstoffatome, Fluor- oder Chloratome, Methyl oder Trifluormethyl, X ein Wasserstoffatom oder Methyl und Y eine Cyanogruppe, Methylcarbonyl, eine Carbonamidgruppe, gegebenenfalls am Amidstickstoff durch ein oder zwei niedere Alkylreste mit 1 bis 3 C-Atomen substituiert, oder eine Alkoxycarbonylgruppe mit 1 bis 3 C-Atomen im Alkyl bedeuten, die wertvolle Zwischenprodukte für chemische Synthesen, insbesondere für Arzneimittel, darstellen.

Es wird darauf hingewiesen, daß die erfindungsgemäßen Verbindungen der Formel I als cis-trans-Isomere Ia und b vorliegen.

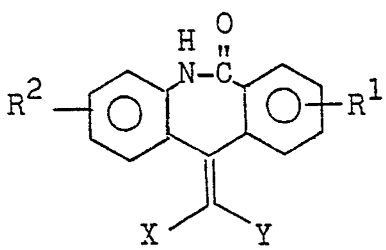

Ia

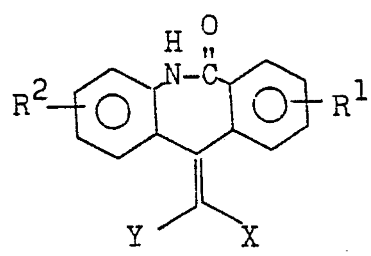

Ib

D/BL

Gegebenenfalls können die cis-trans-Isomeren beispielsweise durch fraktionierte Kristallisation oder durch Säulenchromatographie in an sich üblicher Weise getrennt werden. Die Zuordnung der einzelnen Isomeren kann beispielsweise durch Röntgenstrukturanalyse, wie aus den Beispielen hervorgeht, erfolgen.

Die Verbindungen der Formel I werden hergestellt durch Carbonyl-Olefinierung, indem man ein 5,6-Dihydromorphantridin-6,11-dion der Formel II

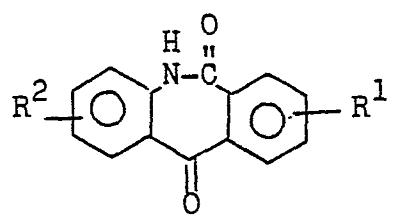

II.

in der $R^1$ und $R^2$ die für Formel I angegebenen Bedeutungen haben, mit einem Phosphonat der Formel IIIa

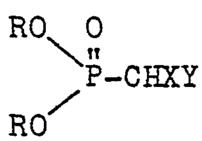

IIIa

in der R einen Alkylrest mit 1 bis 3 C-Atomen und X und Y die für Formel I angegebenen Bedeutungen haben, unter den Bedingungen der Wittig-Horner-Reaktion in einem inerten Lösungsmittel, besonders bevorzugt Dimethylformamid, in Gegenwart von einem Moläquivalent einer Base, bevorzugt Natriumalkoolat, Natriumhydrid oder Natriumamid, bei Temperaturen von 20 bis 80°C umsetzt
oder mit einem Phosphoniumsalz der Formel IIIb

$$\begin{array}{c} Ph \\ | \\ Ph-\overset{\oplus}{P}-CHXY \quad Cl^{\ominus} \\ | \\ Ph \end{array} \qquad IIIb,$$

in der Ph für einen Phenylrest steht und X und Y die für Formel IIIa angegebenen Bedeutungen haben, unter den Bedingungen der klassischen Wittig-Reaktion in einem aprotischen organischen Lösungsmittel, insbesondere einem gesättigten aliphatischen oder gesättigten cyclischen Äther, wie Diäthyläther, Tetrahydrofuran oder Dioxan, oder vorzugsweise in Dimethylformamid, in Gegenwart von einem Moläquivalent einer Base, insbesondere einem Alkalialkoholat, vorzugsweise Natriummethylat oder Natriumäthylat oder Natriumhydrid, Natriumamid oder einer metallorganischen Verbindung, wie Butyllithium, bei Temperaturen von 20 bis 100°C umsetzt.

Interessanterweise führen die genannten Umsetzungen an dem substituierten heterocyclischen 7-Ring in glatter Reaktion zu den erfindungsgemäßen Verbindungen der Formel I.

Eine weitere Herstellungsmöglichkeit für erfindungsgemäße Verbindungen der Formel I, in denen Y, wie oben angegeben, einen gegebenenfalls substituierten Carbonamidrest darstellt, besteht darin, daß man 5,6-Dihydro-11-carbalkoxymethylen-morphantridin-6-on der Formel I, in der Y eine -COOR-Gruppe, wobei R einen niedrigen Alkylrest mit 1 bis 3 C-Atomen bedeutet, mit alkoholischer Alkalilauge bei Temperaturen von 40 bis 90°C in an sich üblicher Weise zur Carbonsäure verseift, das erhaltene 5,6-Di-

BAD ORIGINAL

hydro-11-carboxymethylen-morphantridin-6-on der Formel I, in der Y den Carboxyrest darstellt, durch Umsetzung mit Thionylchlorid in einem inerten Lösungsmittel bei Temperaturen von 20 bis 80°C in das Carbonsäurechlorid überführt und dieses durch Umsetzung mit Ammoniak oder einem Amin der Formel $-N\genfrac{}{}{0pt}{}{R}{R}$ , in der die Reste R niedrig Alkyl mit 1 bis 3 C-Atomen bedeuten, zweckmäßig in wäßrigem Medium oder in einem inerten organischen Lösungsmittel, wie einem cyclischen gesättigten Äther, insbesondere Tetrahydrofuran oder Dioxan, zweckmäßig bei Temperaturen von 50 bis 90°C in das entsprechende 5,6-Dihydro-11-carboxamidomethylen-morphantridin-6-on überführt.

Die 5,6-Dihydro-morphantridin-6,11-dione der Formel II sind teilweise literaturbekannt (F. Hunziker et al., Helv. Chim. Acta 49, 1433-1439 (1966); L.H. Werner et al., J. Med. Chem. 8, 74-80 (1965); G. Caronna et al., Gazz. chim. ital. 84, 1135-1140 (1954)) oder sind aus den entsprechenden Anthrachinonen durch Ringerweiterung mit Hilfe der Schmidt-Reaktion, wie es in den Beispielen beschrieben wird, oder durch Halogensubstitution des Grundkörpers (E. Hardtmann u. H. Ott, J. Org. Chem. 34, 2244-2248 (1969)) zugänglich.

Die erfindungsgemäßen Verbindungen der Formel I sind wertvolle Zwischenprodukte bei der Synthese pharmakologisch wirksamer Verbindungen.

Durch Umsetzung der Verbindungen der Formel I in an sich üblicher Weise mit überschüssigem Phosphoroxychlorid, das gleichzeitig als Lösungsmittel dient, und gegebenenfalls in Gegenwart einer katalytischen Menge N,N-Dimethylanilin unter 3 bis 6 stündigem Erhitzen auf Rückflußtemperaturen wird das entsprechende Iminochlorid der Formel IV nach dem Abdestillieren des überschüssigen Phosphoroxychlorids

BAD ORIGINAL

und Aufarbeitung in einem wäßrigen zweiphasigen System durch Extraktion mit einem chlorierten Kohlenwasserstoff, wie Chloroform oder Methylenchlorid, isoliert.

Aus dem Iminochlorid der Formel IV

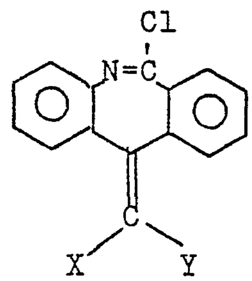

IV

können Verbindungen der Formel V

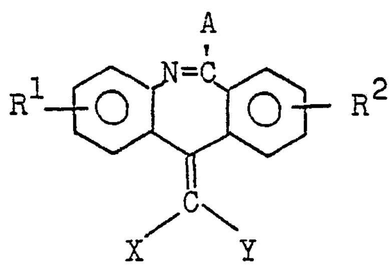

V

hergestellt werden, in denen $R^1$, $R^2$, X und Y die für Formel I angegebenen Bedeutungen haben und A den Rest $-OR^3$ bedeutet, in dem für $R^3$ ein Aminoalkylrest mit 2 oder 3 C-Atomen im Alkyl und am Aminstickstoff durch Methyl oder Äthyl disubstituiert oder ein N-Methyl-piperidinomethylrest, gegebenenfalls in Form des N-Oxids, steht oder A den Aminorest $-N{<}\begin{matrix}R^4\\R^5\end{matrix}$ bedeutet, der für Piperidin, Piperazin oder Homopiperazin, an den Ringkohlenstoffatomen gegebenenfalls durch Methyl oder Hydroxyl und am gegebenenfalls vorhandenen Ringstickstoffatom durch Methyl, Äthyl, ß-Hydroxyäthyl, Cyclopropyl oder Propinyl substituiert und gegebenenfalls in Form des N-Oxids vorliegend, steht oder in dem einer der Reste $R^4$ oder $R^5$

Wasserstoff und der andere einen Aminoalkylrest mit 2 oder 3 C-Atomen im Alkyl und am Aminstickstoff durch Methyl oder Äthyl disubstituiert oder ein N-Methyl-piperidinomethylrest, gegebenenfalls in Form des N-Oxids, bedeutet, durch Umsetzung mit einem Nukleophil AH, in dem A die oben angegebenen Bedeutungen hat, zweckmäßig in Gegenwart einer überschüßigen Menge des verwendeten Amins oder Alkohols AH, das gleichzeitig als Lösungsmittel und gegebenenfalls als säurebindendes Mittel dient. Gegebenenfalls kann in Gegenwart eines inerten Lösungsmittels, wie einem cyclischen gesättigten Äther, insbesondere Tetrahydrofuran oder Dioxan, von Benzol oder eines Benzolkohlenwasserstoffes, wie Xylol, Mesitylen oder Decahydronaphthalin gearbeitet werden. Die Umsetzung erfolgt in der Regel bei Temperaturen von 80 bis 150°C, bevorzugt 90 bis 120°C, und ist im allgemeinen innerhalb von 3 bis 10 Stunden beendet. Gegebenenfalls ist der Ausschluß des Sauerstoffs der Luft und das Arbeiten unter Inertgas, beispielsweise unter Stickstoff, von Vorteil.

Bei den Umsetzungen wird das Nukleophil AH vorteilhafterweise in mindestens 2-fachem molarem bis 20 molaren Überschuß verwendet.

Die freien 6-substituierten 11-Alkylen-morphantridine der Formel V können gegebenenfalls in üblicher Weise in das N-Oxid und/oder in das Säureadditionssalz einer pharmakologisch verträglichen Säure überführt werden. Pharmazeutisch verträgliche Säuren sind beispielsweise Salzsäure, Maleinsäure und Methansulfonsäure.

Die Verbindungen der Formel V, ihre reinen cis-trans-Isomeren und ihre Säureadditionssalze mit einer pharmakologisch verträglichen Säure weisen wertvolle pharmakologische Eigenschaften auf. Sie können aufgrund ihrer pharmakodynamischen Eigenschaften insbesondere als Neuroleptika, Sedativa, Hypnotika, Antiparkinson-Mittel, Analgetika oder Antidepressiva Verwendung finden.

Therapeutische Mittel mit üblichen Trägerstoffen oder Verdünnungsmitteln und den üblicherweise verwendeten technischen Hilfsstoffen können entsprechend der gewünschten Applikationsart und mit einer zur Anwendung geeigneten Dosiereinheit in an sich üblicher Weise hergestellt werden. Als Einzeldosen beim Menschen kommen 10 bis 100 mg in Betracht.

Beispiele für Verbindungen der Formel V sind:

cis,trans-11-Cyanmethylen-6-(4-methyl-piperazin-1-yl)-morphantridin,
cis-11-Cyanmethylen-6-(4-methyl-piperazin-1-yl)-morphantridin,
trans-11-Cyanmethylen-6-(4-methyl-piperazin-1-yl)-morphantridin,
cis,trans-11-Cyanmethylen-6-(4-methyl-4-oxy-piperazin-1-yl)-morphantridin,
cis-11-Cyanmethylen-6-(4-methyl-4-oxy-piperazin-1-yl)-morphantridin,
trans-11-Cyanmethylen-6-(4-methyl-4-oxy-piperazin-1-yl)-morphantridin,

cis,trans-11-Cyanmethylen-2-methyl-6-(4-methyl-piperazin-1-yl)-morphantridin,
cis,trans-11-Cyanmethylen-3-methyl-6-(4-methyl-piperazin-1-yl)-morphantridin,
cis,trans-11-Cyanmethylen-6-(4-äthyl-piperazin-1-yl)-morphantridin,
cis,trans-11-Cyanmethylen-6-(2-piperidin-1-yl-äthylamino)-morphantridin,
cis,trans-11-Cyanmethylen-6-(N-methyl-piperidin-3-yl-methoxy)-morphantridin,
cis,trans-11-Cyanmethylen-6-(N'-methyl-homopiperazin-1-yl)-morphantridin.

Die Erfindung wird durch die folgenden Beispiele näher erläutert:

I. Herstellung von erfindungsgemäßen Verbindungen

<u>Beispiel 1</u>

cis,trans-11-Cyanmethylen-5,6-dihydro-morphantridin-6-on

a) 30,0 g (135 mM) 5,6-Dihydro-morphantridin-6,11-dion werden in 300 ml Dimethylformamid gelöst und unter Stickstoff gerührt. Man läßt dann gleichzeitig 35,4 g (200 mM) Diäthyl-cyanmethyl-phosphonat und 35,0 g (200 mM) Natriummethylat (30 %) gelöst in 100 ml Dimethylformamid langsam zutropfen (Farbzunahme und Temperaturerhöhung zeigen den Beginn der Wittig-Reaktion an). Nach 12-stündigem Nachrühren bei Raumtemperatur gießt man das Reaktionsprodukt auf Eiswasser und saugt die ausfallenden Festkörper ab. Nach gutem Nachwaschen mit Wasser wird das

Rohprodukt getrocknet und aus Äthanol umkristallisiert. Ausbeute: 32,5 g (98 %) 11-Cyanmethylen-5,6-dihydro-morphantridin-6-on, farblose Kristalle mit Schmp. 221-223°C.

b) Klassisches Wittig-Verfahren: Man legt Triphenyl-cyanomethyl-phosphonium-chlorid in Dimethylformamid vor, läßt anschließend 1 Moläquivalent einer 30 %igen Natriummethylat-Lösung zutropfen oder versetzt mit 1 Moläquivalent Natriumhydrid und fügt zuletzt 1 Moläquivalent einer Lösung von 5,6-Dihydro-morphantridin-6,11-dion in Dimethylformamid hinzu. Man läßt das Reaktionsgemisch 5 bis 8 Stunden bei 50 bis 80°C nachrühren, gießt anschließend auf Eiswasser und extrahiert mehrmals mit Methylenchlorid. Nach dem Trocknen und Einengen der organischen Phase kristallisiert man das Rohprodukt aus Äthanol um. Ausbeute: 67 % farblose Kristalle vom Schmp. 220 bis 222°C.

Beispiel 2

cis,trans-11-Carbomethoxymethylen-5,6-dihydro-morphantridin-6-on

cis,trans-11-Carbomethoxymethylen-5,6-dihydro-morphantridin-6-on wird gemäß Beispiel 1a aus 5,6-Dihydro-morphantridin-6,11-dion und Diäthyl-carbomethoxymethyl-phosphonat als Wittig-Horner-Reagens hergestellt: Ausbeute 96 %, Schmp. 184-185°C.

Beispiel 3

cis,trans-11-Carboxamidomethylen-5,6-dihydro-morphantridin-6-on

a) cis,trans-11-Carboxamidomethylen-5,6-dihydro-morphantridin-6-on wird nach Beispiel 1a unter Verwendung von Diäthyl-phosphonoacetamid (hergestellt durch Arbuzov-Reaktion aus Triäthylphosphit und Chloracetamid) unter Erhöhung der Nachrührtemperatur auf 50 bis 80°C hergestellt; an Stelle von Natriummethylat setzt man vorteilhaft Natriumhydrid, suspendiert in DMF, ein: Schmp. 283-288°C.

b) 1. 20 g (72 mM) 11-Carbomethoxymethylen-5,6-dihydro-morphantridin-6-on (Verbindung Beispiel 2) in 20 ml Äthanol werden mit 20 ml 10 %iger Natronlauge versetzt, kurz auf 60°C erwärmt und anschließend 2 Stunden bei Raumtemperatur gerührt. Nach dem Filtrieren stellt man das Filtrat mit 10 %iger Salzsäure sauer und saugt die ausfallenden Kristalle unter gutem Nachwaschen mit Wasser ab. Man isoliert 19,0 g (99 %) 5,6-Dihydro-morphantridin-6-on-11-methylen-carbonsäure mit Schmp. 273-275°C.

2. 6,0 g (23 mM) 5,6-Dihydro-morphantridin-6-on-11--methylen-carbonsäure werden mit 80 ml Thionylchlorid versetzt und bei Raumtemperatur gerührt. Innerhalb 1 Stunde tritt Lösung ein. Nach weiterem einstündigem Rühren zieht man das Thionylchlorid im Ölpumpenvakuum ab, nimmt den Rückstand mit wenig Toluol auf und zieht das Lösungsmittel wiederum vollständig ab. Das verbleibende 5,6-Dihydro-morphantridin-6-on-11--methylen-carbonsäurechlorid ist genügt rein für die weitere Umsetzung: Man nimmt den Rückstand in 200 ml conz. Ammoniak auf, gibt unter Rühren bis zur Lösung Äthanol hinzu und erwärmt 2 bis 3 Stunden auf 90°C. Nach dem Abkühlen

engt man das Volumen auf 1/4 ein und saugt die ausgefallenen Festkörper ab. Man isoliert 4,8 g (79 %) 11-Carboxamidomethylen-5,6-dihydro-morphantridin-6-on mit Schmp. 284-288°C.

Beispiel 4

cis,trans-11-N-Methylcarboxamidomethylen-5,6-dihydro-morphantridin-6-on

a) Herstellung analog Beispiel 3a unter Verwendung von Diäthyl-N-methyl-phosphonoacetamid (hergestellt durch Arbuzov-Reaktion aus Triäthylphosphit und N-Methylchloracetamid): Schmp. 251-254°C.

b) Herstellung analog Beispiel 3b: 5,0 g (18 mM) 5,6-Dihydro-morphantridin-6-on-11-methylencarbonsäurechlorid werden mit 200 ml 40 %iger wäßriger Methylamin-Lösung versetzt und 2 Stunden bei 80 bis 90°C gerührt. Nach der Aufarbeitung wie oben isoliert man 4,7 g (94 %) 11-N-Methylcarboxamidomethylen-5,6-dihydro-morphantridin-6-on mit Schmp. 250 bis 253°C.

Beispiel 5

cis,trans-11-N,N-Dimethyl-carboxamidomethylen-5,6-dihydro-morphantridin-6-on

a) Herstellung analog Beispiel 3a unter Verwendung von Diäthyl-N,N-dimethyl-phosphonoacetamid (hergestellt durch Arbuzov-Reaktion aus Triäthylphosphit und N,N-Dimethylchloracetamid): Schmp. 88-94°C.

b) Herstellung analog Beispiel 4b unter Verwendung einer 40 %igen wäßrigen Dimethylamin-Lösung:

11-N,N-Dimethyl-carboxamidomethylen-5,6-dihydro--morphantridin-6-on: Schmp. 89-94°C.

Beispiel 6

cis,trans-5,6-Dihydro-11-methylcarbonyl-methylen-morphantridin-6-on

Herstellung analog Beispiel 1a unter Verwendung von Dimethyl-2-oxopropyl-phosphonsäureester als Wittig-Horner Reagens. Schmp. 167-168°C.

Beispiel 7

cis,trans-11-(α-Methyl)-cyanmethylen-5,6-dihydro-morphantridin-6-on

Die Herstellung von 11-(α-Methyl)-cyanmethylen-5,6-dihydro-morphantridin-6-on erfolgt analog Beispiel 1a durch Carbonyl-Olefinierung mit Diäthyl-1-cyano-äthyl-phosphonat (zugänglich durch Arbuzov-Reaktion aus Triäthylphosphit und 2-Brom-propionitril oder nach D.L. Comins et al., Synthesis (1978), 309) vorteilhaft unter Verwendung von Natriumhydrid anstelle des Natriumäthylats während 4 bis 6 Stunden bei 80°C: Schmp. 256-260°C.

Beispiel 8

cis,trans-9-Chlor-11-cyanmethylen-5,6-dihydro-morphantridin-6-on

a) 9-Amino-5,6-dihydro-morphantridin-6,11-dion

20,0 g (90 mM) 2-Aminoanthrachinon werden in einer Mischung aus 96 ml conz. Schwefelsäure und 32 ml

Methylenchlorid eingetragen und unter Rühren bei Raumtemperatur gelöst. In diese Reaktionsmischung gibt man bei 20°C (Kühlung von außen durch ein Wasserbad) portionsweise während 5 Stunden 6,8 g (105 mM) Natriumazid hinzu. Man läßt über Nacht bei Raumtemperatur rühren und gießt dann die Reaktionsmischung vorsichtig auf 3 l Eiswasser. Man stellt den pH-Wert der wäßrigen Mischung mit conz. Natronlauge auf 9, saugt die ausfallenden Festkörper ab und wäscht mit reichlich Wasser nach. Das Rohprodukt wird im Vakuum-Trockenschrank bei 70°C getrocknet.

Zur Trennung des Isomerengemisches - es handelt sich nach Ausweis des 270 MHz $^{1}$H-NMR-Sprektrums um 4 Aminoisomere - digeriert man in 1 l siedendem Äthanol und saugt den unlöslichen Anteil (ca. 1/4 der Gesamtmenge) in der Hitze ab. Man isoliert 4,5 g (21 %) stark angereichertes 9-Amino-5,6-dihydro-morphantridin-6,11-dion vom Schmp. 295-297°C; Umkristallisieren aus ca. 200 ml Äthanol/Dimethylformamid 3 : 1 unter Verwendung von Aktivkohle liefert das reine Isomere. Die Stellung der Amino-Gruppe ergiebt sich aus der Röntgenstrukturanalyse des entsprechenden Endproduktes der Formel V (Beispiel 24).

$^{1}$H-NMR (270 MHz, $D_6$DMSO): $\delta$= 6,30 (s; $NH_2$), 6,97 (d; 1H), 7,01 (s; 1H), 7,20 (t, 1H), 7,37 (d, 1H), 7,59 (t, 1H), 7,72 (d, 1H), 7,98 (d, 1H), 10,70 (s, NH).

Durch fraktionierte Kristallisationen der äthanolischen Mutterlauge kann man die noch verbleibenden 2-, 3- und 8-Amino-5,6-dihydro-morphantridin-6,11-dione anreichern (Analyse der Fraktionen jeweils durch 270 MHz $^{1}$H-NMR Aufnahmen).

b) 9-Chlor-5,6-dihydro-morphantridin-6,11-dion

3,0 g (12,6 mM)9-Amino-5,6-dihydro-morphantridin-6,11-dion werden in eine Mischung aus 120 ml Wasser und 120 ml conz. Salzsäure eingetragen. Bei 0 bis 5°C tropft man unter gutem Rühren eine Lösung von 0,87 g (12,6 mM) Natriumnitrit in 10 ml $H_2O$ hinzu und läßt 2,5 Stunden bei einer Temperatur zwischen 0 und 5°C nachrühren. Anschließend gibt man zur Zerstörung der überschüssigen salpetrigen Säure etwas Harnstoff hinzu und fügt darauf 120 mM eines frisch hergestellten Cu-I-Chlorid-Katalysators in conz. Salzsäure hinzu (Stickstoffentwicklung). Man läßt 30 Minuten bei Raumtemperatur nachrühren und erhitzt anschließend 1 Stunde auf 100°C unter beständigem Rühren. Das Reaktionsgemisch wird nach dem Abkühlen auf Eiswasser gegossen und dreimal mit je 300 ml Methylenchlorid extrahiert. Die organische Phase wird anschließend mit Wasser gewaschen, getrocknet und eingeengt. Man isoliert 1,9 g 9-Chlor-5,6-dihydro-morphantridin-6,11-dion vom Schmp. 265-267°C.

c) Weiterreaktion zum Endprodukt analog Beispiel 1:

cis,trans-9-Chlor-11-cyanmethylen-5,6-dihydro-morphantridin-6-on: Schmp. 250-255°C.

Beispiel 9

cis,trans-2(3,8 bzw. 9)-Chlor-11-cyanmethylen-5,6-dihydro-morphantridin-6-on

Das als Ausgangsmaterial eingesetzte, durch Ringerweiterung von 2-Chlor-anthrachinon nach L.H. Werner et al., J. Med. Chem. 8, 74 (1965) erhaltene Monochlor-5,6-dihydro--morphantridin-6,11-dion-Isomerengemisch (im wesentlichen sind 3 verschieden chlorierte Isomere vorhanden) konnte im Gegensatz zur Aussage von L.H. Werner et al., J. Med. Chem. 8, 74 (1965) durch fraktionierte Kristallisation nicht getrennt werden. (Es wurden zwar Fraktionen mit ähnlichem Schmp. wie in L.H. Werner et al., J. Med. Chem. 8, 74 (1965) angegeben erhalten, die aber nach Ausweis des 270 MHz $^1H$-NMR-Spektrums Gemische aus 2 bis 3 Isomeren waren.) Deshalb wurde mit dem Isomerengemisch weitergearbeitet und erst beim entsprechenden Endprodukt der Formel V (Beispiel 25) eine Separierung vorgenommen. Die Carbonyl-Olefinierung (Beispiel 1) liefert ein 2(3,8 bzw. 9)-Chlor-11-cyanmethylen-5,6-dihydromorphantridin-6-on--Isomerengemisch mit Schmp. 148-151°C.

Beispiel 10

cis,trans-4-Chlor-11-cyanmethylen-5,6-dihydro-morphantridin-6-on

Als Ausgangsmaterial diente das nach L.H. Werner et al., J. Med. Chem. 8, 74 (1965) hergestellte Ringerweiterungsprodukt aus 1-Chloranthrachinon, das durch Säulenchromatographie (Kieselgel, Methylenchlorid/Methanol 95/5) in die polare Komponente (Dünnschicht: Kieselgel, Toluol/Methanol 85/15)Die 4-Stellung des Chlors ist nicht gesichert. Die Wittig-Reaktion (Beispiel 1) ergibt das 4-Chlor-11--cyanmethylen-5,6-dihydro-morphantridin-6-on mit Schmp. 231-233°C.

Beispiel 11

cis,trans-7-Chlor-11-cyanmethylen-5,6-dihydro-morphantridin-6-on

Als Ausgangsmaterial dient die unpolare Komponente (Dünnschicht: Kieselgel, Toluol/Methanol 85/15) des Monochlor-5,6-dihydro-morphantridin-6,11-dions (siehe Beispiel 10) mit Schmp. 269-270°C.

Die 7-Stellung des Chlors ist nicht gesichert. Die Carbonyl-Olefinierung (Beispiel 1) ergibt das 7-Chlor-11-cyanmethylen-5,6-dihydro-morphantridin-6-on mit Schmp. 207-210°C.

Beispiel 12

cis,trans-2-Chlor-11-cyanmethylen-5,6-dihydro-morphantridin-6-on

Als Ausgangsmaterial dient 2-Chlor-5,6-dihydro-morphantridin-6,11-dion (E. Hardtmann u. H. Ott, J. Org. Chem. 34, 2244-2248 (1969)).

Die Carbonyl-Olefinierung gemäß Beispiel 1 ergibt das 2-Chlor-11-cyanmethylen-5,6-dihydro-morphantridin-6-on mit Schmp. > 270°C.

Beispiel 13

cis,trans-9-Fluor-11-cyanmethylen-5,6-dihydro-morphantridin-6-on

a) 3,6 g (15,1 mM) 9-Amino-5,6-dihydro-morphantridin-6,11-dion (Beispiel 8a) wird in einer Mischung aus 100 ml Wasser und 100 ml conz. Salzsäure suspendiert. Nach Kühlen auf 0 bis 5°C wird dann unter gutem Rühren eine Lösung von 1,06 g (15,1 mM) Natriumnitrit in 20 ml Wasser zugetropft. Die gelbe Reaktionsmischung wird anschließend 2 Stunden bei 0 bis

$5^{\circ}C$ nachgerührt. Anschließend fügt man 100 ml 50 %ige Tetrafluorborsäure hinzu und läßt 1 Stunde bei 0 bis $5^{\circ}C$ nachrühren.

Der Niederschlag wird abgesaugt und mit reichlich Wasser ausgewaschen. Nach dem Trocknen an der Luft wird das Diazoniumtetrafluoroborat (4,8 g) in einem Zweihalskolben mit Rückflußkühler im leichten Stickstoffstrom erhitzt. Bei ca. $110^{\circ}C$ Badtemperatur setzt die Reaktion ein. Man erhöht nach Abklingen der Reaktion die Badtemperatur 15 Minuten lang noch auf $200^{\circ}C$. Nach Abkühlen kocht man die Festkörper zur Reinigung dreimal in je 50 ml Methanol aus und saugt in der Hitze ab. Aus den Methanol-Mutterlaugen kristallisiert noch ein weiterer Teil des Produktes aus.

Man isoliert insgesamt 3,2 g cis,trans-5,6-Dihydro-9-fluor-morphantridin-6,11-dion vom Schmp. 250 bis $254^{\circ}C$.

b) Weiterreaktion zum Endprodukt analog Beispiel 1:

cis,trans-9-Fluor-11-cyanmethylen-5,6-dihydro-morphantridin-6-on: Schmp. 280-$285^{\circ}C$.

Beispiel 14

cis,trans-3-Methyl-11-cyanmethylen-5,6-dihydro-morphantridin-6-on

Als Ausgangsmaterial dient das nach L.H. Werner et. al., J. Med. Chem. 8, 74 (1965) hergestellte Ringerweiterungsprodukt aus 2-Methyl-anthrachinon, das durch fraktionierte Kristallisation aus Toluol (schwerer lösliche

Fraktion) und Umkristallisation aus Dimethylformamid in 3-Methyl-5,6-dihydro-morphantridin-6,11-dion mit Schmp. 259 bis 263°C separiert wird.

Die Carbonylolefinierung (Beispiel 1) liefert cis,trans-3-Methyl-11-cyanmethylen-5,6-dihydro-morphantridin-6-on mit Schmp. 233-235°C.

Beispiel 15

cis,trans-2-Methyl-11-cyanmethylen-5,6-dihydro-morphantridin-6-on

Als Ausgangsmaterial dient das in Toluol als leichter lösliche Fraktion (siehe Beispiel 14) erhaltene und durch Umkristallisation aus Dioxan/Äthanol 1 : 2 angereicherte 2-Methyl-5,6-dihydro-morphantridin-6,11-dion mit Schmp. 198-202°C.

Durch weitere fraktionierte Kristallisation der Dioxan/Äthanol-Mutterlauge kann man auch eines der beiden restlichen noch vorliegenden 8- bzw. 9-Methyl-5,6-dihydro-morphantridin-6,11-dione anreichern.

Die Carbonyl-Olefinierung liefert das cis,trans-2-Methyl-11-cyanmethylen-5,6-dihydro-morphantridin-6-on mit Schmp. 228-230°C.

Beispiel 16

cis,trans-2(3,8 bzw. 9)-Trifluormethyl-11-cyanmethylen-5,6-dihydro-morphantridin-6-on

Die Herstellung des Ausgangsmaterials 2-Trifluormethyl-anthrachinon ist in dem DRP 713 745 beschrieben.

Die Ringerweiterung zu den vier (2,3,8 bzw. 9)-Trifluormethyl-5,6-dihydro-morphantridin-6,11-dion-Isomeren wird analog Beispiel 8a durchgeführt. Umkristallisieren aus Toluol liefert das Isomerengemisch mit Schmp. 177-179°C. Durch fraktionierte Kristallisation aus Äthanol kann man die einzelnen Isomeren anreichern. Mit der größten Ausbeute kristallisiert ein Trifluormethyl-5,6-dihydro-morphantridin-6,11-dion-Isomeres mit Schmp. 230-234°C.

Weiterreaktion zum Endprodukt analog Beispiel 1: cis,trans-2(3,8 bzw.9)-Trifluormethyl-11-cyanmethylen-5,6-dihydro-morphantridin-6-on-Isomerengemisch: Schmp. 130-133°C.

II. Herstellung von Verbindungen der allgemeinen Formel V

Beispiel 17

cis- und trans-11-Cyanmethylen-6-(4-methyl-piperazin-1-yl)-morphantridin

a) 20,0 g (81 mM) 11-Cyanmethylen-5,6-dihydro-morphantridin-6-on (cis,trans-Isomerengemisch) wird mit 160 ml Phosphoroxychlorid und 3,5 ml N,N-Dimethylanilin versetzt und 4 Stunden unter Stickstoffatmosphäre am Rückfluß gekocht. Nach vollständigem Abdestillieren des überschüssigen Phosphoroxychlorids und Dimethylanilins am Ölpumpenvakuum verteilt man den Rückstand zwischen Methylenchlorid und Wasser, extrahiert die wäßrige Phase noch zweimal mit Methylenchlorid und wäscht die vereinigten organischen Phasen mit verdünnter HCl und Wasser gründlich nach. Trocknen und Einengen der organischen Phase liefert

20,8 g (97 %) 6-Chlor-11-cyanmethylen-morphantridin, das für die weitere Umsetzung genügend rein ist.

20,8 g (79 mM) 6-Chlor-11-cyanmethylen-morphantridin wird mit 60 ml N-Methyl-piperazin versetzt und 3 bis 5 Stunden bei 110°C unter Stickstoff gerührt. Das dunkle homogene Reaktionsgemisch gießt man dann nach dem Abkühlen auf Eiswasser und saugt das gelbliche Rohprodukt, 11-Cyanmethylen-6-(4-methyl-piperazin-1-yl)-morphantridin, ab. Nach dem Trocknen im Vakuumtrockenschrank kristallisiert man das Rohprodukt aus Äthanol unter Zusatz von Aktivkohle um. Man erhält 19,5 g (75 %) gelbes 11-Cyanmethylen-6-(4-methyl-piperazin-1-yl)-morphantridin in Form eines cis,-trans-Isomerengemisches mit dem Schmp. 148-150°C.

Zur Trennung der cis, trans-Isomeren digeriert man das Isomerengemisch in ca. 80 ml siedendem Methanol und saugt die unlöslichen Anteile in der Hitze ab. Man erhält 3,1 g gelbe Festkörper, die nach Ausweis des Dünnschichtchromatogramms (Kieselgel, Laufmittel Toluol/Methanol 85/15) vornehmlich aus dem unpolaren Isomeren a bestehen. Nach dem Einengen des Filtrats nimmt man den Rückstand in wenig siedendem Methylenchlorid auf, bis gerade alles in Lösung geht. Beim Abkühlen kristallisieren 3,0 g gelbes Produkt aus, das schnell abgesaugt und mit sehr wenig eiskaltem Methylenchlorid nachgewaschen wird. Die Dünnschichtchromatographie zeigt eine sehr gute Anreicherung des polaren Isomeren b.

Durch mehrfache Wiederholung dieser beiden aufeinanderfolgenden Operationen erhält man insgesamt je ca. 10 bis 11 g Fraktionen der stark angereicherten cis,

trans-Isomeren a und b, die dann noch 1 bis 2 mal aus Äthanol umkristallisiert werden.

Das reine Isomere a fällt in Form von gelben, rechteckigen Plättchen mit Schmp. 210 bis 212°C, das reine Isomere b in Form von gelben spitzen Nadeln mit Schmp. 182 bis 184°C. an.

Die Röntgenstrukturanalyse zeigt, daß es sich bei a um das cis-isomere und bei b um das trans-isomere 11-Cyanmethylen-6-(4-methyl-piperazin-1-yl)-morphantridin handelt.

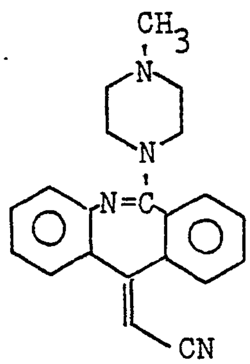

a

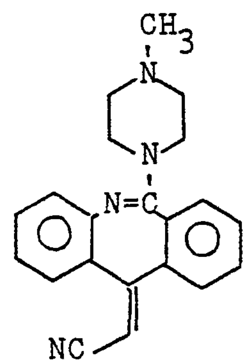

b

Beispiel 18

cis,trans-11-Carbomethoxymethylen-6-(4-methyl-piperazin-1--yl)-morphantridin

Herstellung analog Beispiel 17 aus 11-Carbomethoxymethylen-5,6-dihydro-morphantridin-6-on (Beispiel 2): Die Reinigung des Rohproduktes erfolgt durch Säulenchromatographie (Kieselgel, Laufmittel Methylenchlorid). Man erhält das cis,trans-Isomerengemisch in 45 % Ausbeute als gelbe Kristalle mit Schmp. 75 bis 79°C.

Beispiel 19

cis,trans-11-Carboxamidomethylen-6-(4-methyl-piperazin-1-yl)-morphantridin

Herstellung analog Beispiel 17 aus 11-Carboxamidomethylen-5,6-dihydro-morphantridin-6-on (Beispiel 3):
Man isoliert nach dem Umkristallisieren aus Äthanol 65 % cis,trans-Isomere in Form von gelben Kristallen mit Schmp. 185-193°C.

Beispiel 20

cis,trans-11-N-Methyl-carboxamidomethylen-6-(4-methyl-piperazin-1-yl)-morphantridin

Herstellung analog Beispiel 17 aus 11-N-Methylcarboxamidomethylen-5,6-dihydro-morphantridin-6-on (Beispiel 4):
Das cis,trans-11-N-Methyl-carboxamidomethylen-6-(4-methyl-piperazin-1-yl)-morphantridin wird mit Hilfe von Säulenchromatographie (Kieselgel, Methylenchlorid/Methanol 95/5 als Laufmittel) gereinigt: gelbe Kristalle mit Schmp. 118- 124°C.

Beispiel 21

cis,trans-11-N,N-Dimethyl-carboxamidomethylen-6-(4-methyl-piperazin-1-yl)-morphantridin . $H_2O$

Herstellung analog Beispiel 20 aus 11-N,N-Dimethyl-carboxamidomethylen-5,6-dihydro-morphantridin-6-on (Beispiel 5):
gelbe Kristalle mit Schmp. 161-163°C.

Beispiel 22

cis,trans-11-Methylcarbonyl-methylen-6-(4-methyl-piperazin-1-yl)-morphantridin . $H_2O$

Herstellung analog Beispiel 20 aus 5,6-Dihydro-11-methylcarbonyl-methylen-morphantridin-6-on (Beispiel 6): gelbe Kristalle mit Schmp. 133-136°C.

Beispiel 23

cis,trans-11-(α-Methyl)-cyanmethylen-6-(4-methyl-piperazin-1-yl)-morphantridin . 1/2 $H_2O$

Herstellung nach Beispiel 17 aus 11-(α-Methyl)-cyanmethylen-5,6-dihydro-morphantridin-6-on (Beispiel 7): Nach der Reinigung mit Hilfe von Säulenchromatographie (Kieselgel, Methylenchlorid/Methanol 95/5) gelbe Kristalle mit Schmp. 96-98°C.

Beispiel 24

cis,trans-9-Chlor-11-cyanmethylen-6-(4-methyl-piperazin-1-yl)-morphantridin

Herstellung nach Beispiel 17 aus 9-Chlor-11-cyanmethylen-5,6-dihydro-morphantridin-6-on (Beispiel 9): Schmp. 90-95°C.

Zur Trennung der cis,trans-Isomeren kristallisiert man fraktioniert aus Äthanol um: als schwerer lösliche Fraktion kristallisiert zuerst das reine cis-Isomere (unpolarere Komponente auf der Dünnschichtplatte: Kieselgel, Laufmittel Toluol/Methanol 85/15) mit dem Schmp. 173-174°C.

Die 9-Stellung des Chlors ist durch Röntgenstrukturanalyse des cis-Isomeren bewiesen.

Beispiel 25

cis,trans 3- bzw. 8-Chlor-11-cyanmethylen-6-(4-methyl-piperazin-1-yl)-morphantridin.

Herstellung nach Beispiel 17 aus dem Monochlor-11-cyanmethylen-5,6-dihydro-morphantridin-6-on-Isomerengemisch des Beispiels 9:
Das im wesentlichen aus 6 Isomeren (Dünnschichtchromatographie, Kieselgel, Toluol/Methanol 85/15, Verdoppelung durch cis,trans-Isomerie) bestehende Endprodukt mit Schmp. 95-99°C wird nach Umkristallisation aus Äthanol mit Hilfe von Säulenchromatographie (Kieselgel, Methylenchlorid/Methanol 95/5) in einzelne Fraktionen angereichert. Dabei konnten als polarer und weniger polarer Anteil die in Beispiel 28 und 24 beschriebenen cis,trans-2- bzw. 9-Chlor-11-cyanmethylen-6-(4-methyl-piperazin-1-yl)-morphantridin-Isomeren isoliert und charakterisiert werden.

Als weitere Fraktionen werden die noch verbleibenden cis,trans-3- bzw. 8-Chlor-11-cyanmethylen-6-(4-methyl-piperazin-1-yl)-morphantridin-Isomeren angereichert. Gelbe Kristalle mit Schmp. 95-98°C.

Beispiel 26

cis,trans-4-Chlor-11-cyanmethylen-6-(4-methyl-piperazin-1-yl)-morphantridin . 1/2 $H_2O$

Herstellung analog Beispiel 17 aus 4-Chlor-11-cyanmethylen-5,6-dihydro-morphantridin-6-on (Beispiel 10):

Nach Säulenchromatographie (Kieselgel, Methylenchlorid/Methanol 95/5) gelbe Kristalle mit Schmp. 90-95°C.

Beispiel 27

cis,trans-7-Chlor-11-cyanmethylen-6-(4-methyl-piperazin-1-yl)-morphantridin

Herstellung analog Beispiel 17 aus 7-Chlor-11-cyanmethylen-5,6-dihydro-morphantridin-6-on (Beispiel 11): gelbe Kristalle mit Schmp. 219-221°C.

Beispiel 28

cis,trans-2-Chlor-11-cyanmethylen-6-(4-methyl-piperazin-1-yl)-morphantridin . 1/2 $H_2O$

Herstellung analog Beispiel 17 aus 2-Chlor-11-cyanmethylen-5,6-dihydro-morphantridin-6-on (Beispiel 12): gelbe Kristalle mit Schmp. 157-162°C.

Beispiel 29

cis,trans-9-Fluor-11-cyanmethylen-6-(4-methyl-piperazin-1-yl)-morphantridin

Herstellung nach Beispiel 17 aus 9-Fluor-11-cyanmethylen-5,6-dihydro-morphantridin-6-on (Beispiel 13): Schmp. 120-125°C.

Beispiel 30

cis,trans-3-Methyl-11-cyanmethylen-6-(4-methyl-piperazin-1-yl)-morphantridin

Herstellung analog Beispiel 17 aus 3-Methyl-11-cyanmethylen-5,6-dihydro-morphantridin-6-on (Beispiel 14): gelbe Kristalle mit Schmp. 192-200°C. Die Position der Methyl-Gruppe in Stellung 3 ist durch Röntgenstrukturanalyse ermittelt.

Zur Trennung der cis,trans-Isomeren kristallisiert man das Isomerengemisch aus Methanol fraktioniert um: man isoliert als erste Fraktion stark angereichertes unpolares (Dünnschicht Kieselgel, Toluol/Methanol 85/15) Isomeres, das nochmals aus Methanol umkristallisiert wird. Dieses Isomere mit dem Schmp. 224°C erweist sich nach Röntgenstrukturanalyse als cis-3-Methyl-11-cyanmethylen-6-(4-methyl-piperazin-1-yl)-morphantridin.

Die entsprechende polare trans-isomere Verbindung erhält man am besten durch fraktionierte Kristallisation des oben anfallenden Mutterlaugen-Rückstandes aus Cyclohexan; das reine trans-3-Methyl-11-cyanmethylen-6-(4-methyl-piperazin-1-yl)-morphantridin schmilzt bei 193-195°C.

Beispiel 31

cis,trans-2-Methyl-11-cyanmethylen-6-(4-methyl-piperazin-1-yl)-morphantridin

Herstellung analog Beispiel 17 aus 2-Methyl-11-cyanmethylen-5,6-dihydro-morphantridin-6-on (Beispiel 15): gelbe Kristalle mit Schmp. 162-164°C. Die Position der Methyl-Gruppe in Stellung 2 ist durch Röntgenstrukturanalyse ermittelt.

Zur Trennung der cis,trans-Isomeren kristallisiert man das Isomerengemisch aus Äthanol fraktioniert um: man isoliert als erste Fraktion stark angereichertes polares

(Dünnschicht Kieselgel, Toluol/Methanol 85/15) Isomeres, das aus Äthanol umkristallisiert wird. Dieses Isomere mit dem Schmp. 183°C erweist sich nach Röntgenstrukturanalyse als trans-2-Methyl-11-cyanmethylen-6-(4-methyl-piperazin-1-yl)-morphantridin . 1/2 $H_2O$.

Die entsprechende unpolare cis-isomere Verbindung erhält man am besten durch mehrmalige Säulenchromatographie (Kieselgel, Laufmittel Methylenchlorid/Methanol 98/2) des oben anfallenden Mutterlaugen-Rückstandes mit Schmp. 92-95°C.

Beispiel 32

cis,trans-2(3,8 bzw. 9)-Trifluormethyl-11-cyanmethylen-6-(4-methyl-piperazin-1-yl)-morphantridin . 0,5 $H_2O$.

Herstellung analog Beispiel 17 aus 2(3,8 bzw. 9)-Trifluormethyl-11-cyanmethylen-5,6-dihydro-morphantridin-6-on (Beispiel 16):
Schmp. 93-96°C.

Beispiel 33

Allgemeine Vorschrift zur Herstellung der Endprodukte der allgemeinen Formel V durch Einführung der verschiedenen nukleophilen Alkylamino- bzw. Alkyloxy-Reste AH in die 6-Stellung der 6-Chlor-morphantridin-Derivate IV.

Das 6-Chlor-morphantridin wird mit 2 bis 5 Äquivalenten des Alkylamins bzw. Aminoalkyl-alkohols AH versetzt und unter Stickstoff 3 bis 5 Stunden auf 110°C erhitzt. Anschließend destilliert man bei Anwesenheit von leichter flüchtigen Aminen bzw. Alkoholen das überschüssige Nukleophil AH im Vakuum ab. In diesen Fällen nimmt man den Rückstand und ansonsten das Reaktionsgemisch in Eiswasser auf und extrahiert mehrmals mit Methylenchlorid. Die vereinigten Methylenchloridphasen werden nach Auswaschen mit Wasser getrocknet und eingeengt. Das verbleibende Rohprodukt wird entweder aus Äthanol unter Zusatz von Aktivkohle umkristallisiert oder (vor allem bei Anwesenheit der höhermolekularen Alkylamine) durch Säulenchromatographie (Kieselgel, Methylenchlorid/Methanol 95/5) gereinigt.

Folgende nach dieser Vorschrift hergestellten Verbindungen der Formel V seien hier aufgeführt:

34. cis,trans-11-Cyanmethylen-6-(4-ß-hydroxyäthyl-piperazin-1-yl)-morphantridin . 1/2 $H_2O$, Schmp. 111-113°C
35. cis,trans-11-Cyanmethylen-6-piperazin-1-yl-morphantridin . $H_2O$, Schmp. 208-211°C
36. cis,trans-11-Cyanmethylen-6-(4-äthyl-piperazin-1-yl)-morphantridin, Schmp. 86-90°C:

Isomerentrennung von cis,trans-11-Cyanmethylen-6-(4-äthyl-piperazin-1-yl)-morphantridin:
Zur Trennung der cis,trans-Isomeren kristallisiert man das Isomerengemisch fraktioniert aus Methanol um: als schwerer lösliche Fraktion kristallisiert zuerst das trans-Isomere (polare Komponente auf der Dünnschichtplatte : Kieselgel, Laufmittel Toluol/Methanol 85/15). Umkristallisieren aus Äthanol liefert das reine trans-Isomere mit Schmp. 181 bis 183°C.

Säulenchromatographie (Kieselgel, Methylenchlorid/Methanol 95/5) des Mutterlaugenrückstandes reinigt auch das unpolarere cis-Isomere mit Schmp. 138 bis 140°C.

37. cis,trans-11-Cyanmethylen-6-homopiperazin-1-yl-morphantridin . HCl . $H_2O$, Schmp. 175-178°C
38. cis,trans-11-Cyanmethylen-6-)2-dimethylamino-äthylamino)-morphantridin . 1/2 $H_2O$, Schmp. 76-79°C
39. cis,trans-11-Cyanmethylen-6-(2-piperidin-1-yl-äthylamino)-morphantridin . 1/2 $H_2O$, Schmp. 83-85°C:

Isomerentrennung von cis,trans-11-Cyanmethylen-6-(2-piperidin-1-yl-äthylamino)-morphantridin:
Die Trennung der cis,trans-Isomeren kann mit Hilfe von Säulenchromatographie vorgenommen werden (Kieselgel, Methylenchlorid/Methanol 95/5): das cis-Isomere (unpolare Komponente auf der Dünnschichtplatte : Kieselgel, Laufmittel Toluol/Methanol 85/15) fällt in Form von gelblichen Kristallen mit Schmp. 76-78°C an, das polarere trans-Isomere schmilzt bei 103-106°C.

40. cis,trans-11-Cyanmethylen-6-(N'-methyl-homopiperazin-1-yl)-morphantridin . 0,75 $H_2O$, Schmp. 73-80°C
41. cis,trans-11-Cyanmethylen-6-(N-methyl-piperidin-3-yl-methoxy)-morphantridin . $H_2O$, Schmp. 93-95°C
42. cis,trans-11-Cyanmethylen-6-(N-methyl-piperidin-2-yl-methoxy)-morphantridin, Schmp. 67-70°C
43. cis,trans-11-Cyanmethylen-6-(N-methyl-piperidin-3-yl-methylamino)-morphantridin . $H_2O$, Schmp. 110-114°C

Beispiel 44

cis,trans-11-Cyanmethylen-6-(4-methyl-4-oxy-piperazin-1--yl)-morphantridin . 2 $H_2O$

3,0 g (9,1 mM) cis,trans-11-Cyanmethylen-6-(4-methyl--piperazin-1-yl)-morphantridin wird in 100 ml heißem

Äthanol gelöst und mit 1,5 ml 30 %igem Wasserstoffperoxid versetzt. Nach 5-stündigem Rückflußkochen zerstört man das überschüssige Wasserstoffperoxid mit Hilfe eines kleinen Platinblechs, das in das Reaktionsgemisch geworfen wird, durch 2-stündiges Rückflußkochen. Nach Filtrieren engt man das Reaktionsgemisch ein und reinigt das erhaltene N-Oxid durch Säulenchromatographie (Kieselgel, Laufmittel Methylenchlorid/Methanol 95/5). Man isoliert 2,5 g (80 %) gelbe Kristalle mit Schmp. 141-148°C.

Zur Trennung der cis,trans-Isomeren kristallisiert man das Isomerengemisch aus wenig Methylenchlorid fraktioniert um: man isoliert als erste Fraktion stark angereichertes unpolares (Dünnschicht, Kieselgel, Toluol/Methanol 85/15) Isomeres, das aus wenig Äthanol umkristallisiert wird. Aus Analogiegründen zu den vorher beschriebenen cis,trans-Isomeren-Analysen ordnet man dieses Isomere mit dem Schmp. 241°C der cis-Reihe zu.

Die entsprechende polare trans-isomere Verbindung mit Schmp. 169°C erhält man durch Säulenchromatographie (Kieselgel, Laufmittel Methylenchlorid/Methanol 95/5) des oben anfallenden Mutterlaugen-Rückstandes.

Vorteilhaft stellt man beide Isomeren direkt durch Oxidation der beiden nach Beispiel 17 getrennten cis- und trans-isomeren 11-Cyanmethylen-6-(4-methyl-piperazin-1-yl)-morphantridine (Beispiel 17) nach der obigen Vorschrift dar, wobei während der Oxidation keine cis,trans-Isomerisierung stattfindet.

Nach den Ergebnissen der pharmakologischen Experimente eignen sich die Verbindungen der Formel V in Anbetracht ihrer sedativen, apomorphinantagonistischen, analgetischen, reserpin-antagonistischen oder anticholinergischen Wirkung als Neuroleptika, Sedativa, Hypnotika, Analgetika, Antidepressiva oder Antiparkinsonmittel.

Zur Wirkungsanalyse fanden folgende Methoden Verwendung:

1. Sedative Wirkung

Gruppen von 4 x 3 oder 8 x 3 weiblichen NMRI-Mäusen erhalten die Substanzen oral appliziert. Die photoelektrische Bestimmung der durch eine neue Umgebung induzierten Orientierungshypermotilität erfolgt 30 min nach der Applikation der Substanzen für eine Dauer von 30 min. Als ED 50 % wird die Dosis bestimmt, welche im Vergleich zu unbehandelten Kontrolltieren eine Abnahme der Orientierungshypermotilität um 50 % bewirkt.

2. Analgetische Wirkung

Die analgetische Wirkung wird mit Hilfe der sogenannten Brennstrahl-Methode nach D'AMOUR und SMITH (1941) bestimmt. Dabei erhalten Gruppen von 10 weiblichen NMRI-Mäusen die Substanzen intraperitoneal appliziert. Die Schmerzreaktion wird 30 min nach der Applikation ausgelöst. Gemessen wird die Reaktionszeit bis zum Wegziehen des Schwanzes nach Bestrahlung mit einem fokusierten Lichtbündel.

Als ED 100 % wird die Dosis bestimmt, welche im Vergleich zu einer Kontrollgruppe die Reaktionszeit um 100 % verlängert.

3. Anticholinergische Wirkung

Gruppen von 10 weiblichen NMRI-Mäusen erhalten Physostigmin in einer letalen Dosis (0,825 mg/kg) subcutan. Die Prüfsubstanzen werden 30 min vor der Physostigminapplikation oral appliziert.

Als ED 50 wird die Substanzdosis bestimmt, welche 50 % der Tiere vor dem Tod durch Physostigmin schützt.

4. Apomorphin-antagonistische Wirkung

An Gruppen von 4 - 6 weiblichen Sprague-Dawley-Ratten werden durch subcutane Applikation von 1,5 mg/kg Apomorphin Kieferbewegungen ausgelöst, die über implantierte Elektroden registriert werden (Mandibulogramm nach KUBACKI, 1978).

Als ED 50 % wird die Dosis bestimmt, welche die Anzahl der Kieferbewegungen im Vergleich zu unbehandelten Kontrolltieren um 50 % reduziert.

5. Akute Toxizität

Gruppen von 5 - 10 weiblichen NMRI-Mäusen erhalten die Substanzen i.p. Als LD 50 wird die Dosis ermittelt, nach welcher 50 % der behandelten Tiere sterben.

In diesen Experimenten (Tab. 1) werden bei den Verbindungen Beispiel 17 (cis-trans-Gemisch und cis-Isomeres), Beispiel 30 (cis-trans-Gemisch und cis-Isomeres), Beispiel 31 (cis-trans-Gemisch), Beispiel 36 (cis-trans--Gemisch) und Beispiel 39 (cis-trans-Gemisch starke sedative Wirkungen beobachtet, die in der Größenordnung

der Referenzsubstanzen Clozapin oder Perlapin liegen oder diese übertreffen.

Eine analgetische Wirkung findet man bei Beispiel 30 (cis--trans-Gemisch und cis-Isomeres). Das cis-Isomere ist deutlich aktiver als Clozapin.

Die am Physostigmin-Antagonismus beobachtete anticholinergische Wirkung tritt besonders bei den Verbindungen Beispiel 17 (cis-trans-Gemisch und trans-Isomeres), Beispiel 31 (cis-trans-Gemisch), Beispiel 36 (cis-trans--Gemisch und trans-Isomeres), Beispiel 37 (cistrans--Gemisch), Beispiel 44 (cis-trans-Gemisch und transIsomeres) in Erscheinung. Bei Beispiel 17 (cis-trans-Gemisch), Beispiel 31 (cis-trans-Gemisch) und Beispiel 36 (cis-trans--Gemisch) ist sie ähnlich wie beim Clozapin im Verein mit stärkeren sedativen Effekten (s.o.) zu beobachten.

Zusammen mit den genannten Effekten tritt bei den meisten Verbindungen auch eine für Neuroleptika typische apomorphin-antagonistische Wirkung auf, die sich ebenfalls bei den Referenzsubstanzen findet. Vergleicht man die pharmakologischen Eigenschaften der vorliegenden cis-trans--Isomerengemische mit denen der einzelnen reinen Isomeren, so ergeben sich überraschenderweise nicht nur quantitative sondern auch qualitative Differenzen, so daß bei verschiedenen Substanzen neuartige und interessante Wirkungskombinationen gefunden werden.

Das Wirkprofil des cis-trans-Gemisches Beispiel 17 ähnelt dem der Referenzsubstanz Clozapin. Es wirkt aber stärker sedativ und anticholinergisch und ist nicht analgetisch aktiv. Die apomorphin-antagonistische Wirkung ist etwas schwächer als beim Clozapin.

Das cis-Isomere von Beispiel 17 ist Träger der sedativen Wirkung bei einer etwa dem Clozapin vergleichbaren anticholinergischen und geringerer apomorphin-antagonistischer Wirkung.

Das trans-Isomere von Beispiel 17 hingegen zeichnet sich besonders durch anticholinergische und apomorphin-antagonistische Wirkung aus. Die sedative Wirkung tritt ganz zurück. Ein derartiger Wirkungstyp ist neu und sowohl von dem des Clozapin als auch des Perlapin eindeutig abzugrenzen.

Auch die trans-Isomeren von Beispiel 30, 36 und 44 wirken nicht oder nur wenig sedativ, stärker anticholinergisch und apomorphin-antagonistisch und sind ebenso wie das trans-Isomere von Beispiel 17 von den jeweiligen Isomerengemischen zu unterscheiden.

Ein weiteres Isomerengemisch Beispiel 30 wirkt bei hoher sedativer Aktivität ( $>$ Clozapin und Perlapin) mäßig anticholinergisch und stärker analgetisch. Verantwortlich für die starke sedative und die starke analgetische Wirkung ist das cis-Isomere. Auch diese Verbindung stellt in der Kombination sedative plus analgetische Wirkung bei fehlender anticholinergischer und schwacher apomorphin-antagonistischer Wirkung im Vergleich zum Clozapin und Perlapin einen neuen Wirkungstyp dar.

Tabelle 1

| Verbindung Nr. | Geometrische Isomerie | Sedative Wirkung | | Analgetische Wirkung | | Apomorphin-Antagonismus | | Anticholinergische Wirkung | | Toxizität LD 50 |
|---|---|---|---|---|---|---|---|---|---|---|
| | | ED 50 % | R.W. 1) | ED 100 % | R.W. | ED 50 % | R.W. | ED 50 % | R.W. | |
| 17 | cis-trans-G. 2) | 2,78 | 1,71 | 46,4 | 0,04 | 22 | 0,37 | 6,26 | 2,25 | 121 |
| | cis-Isomeres | 1,98 | 2,39 | 31,6 | 0,07 | 46 | 0,17 | 17,4 | 0,81 | 147 |
| | trans-Isomeres | >21,5 | < 0,22 | > 21,5 | < 0,10 | 12 | 0,67 | 4,7 | 3,00 | 332 |
| 30 | cis-trans-G. | 0,804 | 5,90 | 5,17 | 0,40 | 35 | 0,23 | 31,2 | 0,45 | 178 |
| | cis-Isomeres | 1,06 | 4,47 | 0,740 | 2,18 | 85 | 0,09 | 100 | 0,14 | 287 |
| | trans-Isomeres | 33,6 | 0,14 | 68,1 | 0,03 | 19 | 0,42 | 12,3 | 1,15 | > 100 |
| 31 | cis-trans-G. | 3,35 | 1,41 | 10,0 | 0,21 | 42 | 0,19 | 2,74 | 5,15 | 50,0 |
| 36 | cis-trans-G. | 3,34 | 1,42 | > 10,0 | < 0,21 | 43 | 0,19 | 7,80 | 1,81 | 100 |
| | trans-Isomeres | >100 | > 0,04 | > 46,4 | < 0,04 | 22 | 0,37 | 5,60 | 2,52 | > 100 |
| 37 | cis-trans-G. | > 21,5 | > 0,22 | 46,4 | 0,04 | >100 | < 0,08 | 5,49 | 2,57 | 69,8 |
| 39 | cis-trans-G. | 1,61 | 2,94 | > 10,0 | < 0,21 | 46 | 0,17 | >10,0 | < 1,41 | 5,35 |
| 44 | cis-trans-G. | 14,3 | 0,33 | > 46,4 | < 0,04 | 85 | 0,09 | 1,98 | 7,12 | 464 |
| | cis-Isomeres | 17,6 | 0,26 | > 46,4 | < 0,04 | >100 | < 0,08 | 100 | 0,14 | 261 |
| | trans-Isomeres | 100 | 0,04 | >100 | < 0,02 | 25 | 0,32 | 3,74 | 3,77 | 559 |
| Clozapin → | | 4,74 | 1,00 | 2,08 | 1,00 | 8 | 1,00 | 14,1 | 1,00 | 215 |
| Perlapin → | | 2,05 | 2,31 | > 21,5 | < 0,10 | 22 | 0,37 | > 21,5 | < 0,66 | 215 |

1) R.W. = Relative Wirksamkeit

2) G. = Gemisch

Patentansprüche

1. Verbindungen der allgemeinen Formel I

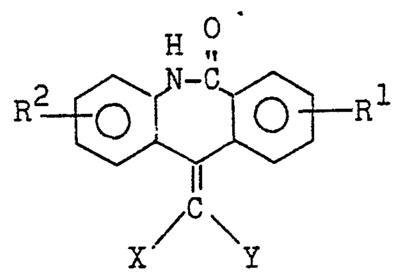

I

in der $R^1$, und $R^2$ Wasserstoffatome, Fluor- oder Chloratome, Methyl oder Trifluormethyl, X ein Wasserstoffatom oder Methyl und Y eine Cyanogruppe, Methylcarbonyl, eine Carbonamidgruppe, gegebenenfalls am Amidstickstoff durch ein oder zwei niedere Alkylreste mit 1 bis 3 C-Atomen substituiert, oder eine Alkoxycarbonylgruppe mit 1 bis 3 C-Atomen im Alkyl bedeuten.

2. cis,trans-11-Cyanmethylen-5,6-dihydro-morphantridin-6-on.

3. cis,trans-11-Carbomethoxymethylen-5,6-dihydro-morphantridin-6-on.

4. cis,trans-11-Carboxamidomethylen-5,6-dihydro-morphantridin-6-on.

5. cis,trans-11-N-Methylcarboxamidomethylen-5,6-dihydro-morphantridin-6-on.

6. cis,trans-11-N,N-Dimethyl-carboxamidomethylen-5,6-dihydro-morphantridin-6-on.

7. cis,trans-5,6-Dihydro-11-methylcarbonyl-methylen--morphantridin-6-on.

8. Verfahren zur Herstellung von Verbindungen der Formel I, dadurch gekennzeichnet, daß man ein 5,6-Dihydromorphantridin-6,11-dion der Formel II

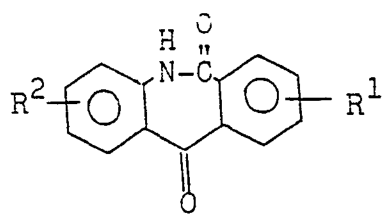

II

in der $R^1$ und $R^2$ die für Formel I angegebenen Bedeutungen haben, mit einem Phosphonat der Formel IIIa

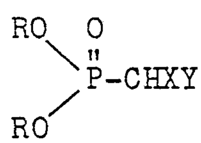

IIIa

in der R einen Alkylrest mit 1 bis 3 C-Atomen und X und Y die für Formel I angegebenen Bedeutungen haben, unter den Bedingungen der Wittig-Horner-Reaktion umsetzt.

9. Verfahren nach Anspruch 8, dadurch gekennzeichnet, daß man die Umsetzung in einem inerten Lösungsmittel in Gegenwart von einem Moläquivalent einer Base bei Temperaturen von 20 bis 80°C durchführt.

BAD ORIGINAL

0019157

Europäisches Patentamt

# EUROPÄISCHER RECHERCHENBERICHT

Nummer der Anmeldung

EP 80 10 2377.1

| Kategorie | EINSCHLÄGIGE DOKUMENTE<br>Kennzeichnung des Dokuments mit Angabe, soweit erforderlich, der maßgeblichen Teile | betrifft Anspruch | KLASSIFIKATION DER ANMELDUNG (Int.Cl.3) |
|---|---|---|---|
| | DE - A - 1 568 217 (BOEHRINGER MANNHEIM)<br>* Anspruch; Formel I *<br>-- | 1,8 | C 07 D 223/20 |
| | DE - A - 2 035 517 (COLGATE-PALMOLIVE)<br>* Anspruch 5 *<br>-- | 8 | |
| A | GB - A - 1 068 744 (K. THOMAE)<br>-- | | |
| A | US - A - 3 993 757 (RICHARDSON-MERRELL)<br>---- | | |

RECHERCHIERTE SACHGEBIETE (Int. Cl.3)

C 07 D 223/20

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 27-08-1980 | FROELICH |

EPA form 1503.1 06.78